Europäisches Patentamt

European Patent Office

Office européen des brevets

(19)

(11) Publication number: **0 136 053**
**A2**

## (12) EUROPEAN PATENT APPLICATION

(21) Application number: **84305664.9**

(22) Date of filing: **20.08.84**

(51) Int. Cl.⁴: **C 12 P 1/00**, C 12 N 5/00
// A61K35/12 ,(C12P1/00,
C12R1:91)

(30) Priority: **20.08.83 JP 150949/83**

(43) Date of publication of application: **03.04.85**
**Bulletin 85/14**

(84) Designated Contracting States: **CH DE FR GB LI NL**

(71) Applicant: **KOKEN LTD., No. 7, Yonban-cho, Chiyoda-ku Tokyo (JP)**
Applicant: **Tajima, Tomoyuki, 5-15, Yawata 6-chome, Ichikawa-shi Chiba-ken (JP)**
Applicant: **Nagasu, Youichiro, No. 9-4, Chuo 3-chome, Yamato-shi Kanagawa-ken (JP)**

(72) Inventor: **Tajima, Tomoyuki, No. 5-15, Yawata 6-chome, Ichikawa-shi Chiba-ken (JP)**

(74) Representative: **Deans, Michael John Percy et al, Lloyd Wise, Tregear & CO. Norman House 105-109 Strand, London WC2R OAE (GB)**

(54) Preparation of proliferation inhibitors.

(57) This invention relates to a method for preparing a proliferation inhibitor for malignant tumour cells of animals including human beings, and the method comprises the steps of cultivating the malignant tumour cells of the animals, afterward transferring them to a culture medium for extraction, adding glucose to the medium, carrying out a cultivation therein, afterward removing the malignant tumour cells therefrom, and collecting the remaining culture medium.

0136053

# PREPARATION OF PROLIFERATION INHIBITORS

## Description

The present invention relates to the preparation of proliferation inhibitors for malignant tumour cells.

We have previously proposed the production of an inhibiting agent against the proliferation of malignant tumour cells comprising the steps of first cultivating malignant tumour cells and then, after removing the malignant tumour cells, extracting an inhibiting agent from the culture medium. We have found that inhibiting agents so produced appear not to show lethal effects against normal cells, but instead to exhibit an inhibiting activity against the proliferation of malignant tumour cells and to have a lethal effect specifically to malignant tumour cells.

The present invention arises from our work seeking to obtain proliferation inhibitors with a significantly enhanced inhibiting effect.

In accordance with the present invention, we provide a method for preparing a proliferation inhibitor for malignant tumour cells, characterised in that it comprises the steps of: cultivating malignant tumour cells; transferring said cells to a culture medium for extraction; adding glucose to said extraction medium; carrying out a cultivation therein; thereafter removing said malignant tumour cells from the culture medium; and collecting said culture medium.

As will be explained below, we have found that by this method we can produce proliferation inhibitors for malignant tumour cells which have a potency several times greater than that obtained by following our previously proposed procedure enabling a significant proliferation inhibiting effect to be produced on malignant tumour cells even in a smaller dose.

The invention is hereinafter more particularly described by way of example only with reference to illustrative detailed examples of procedures in accordance with the present invention.

Example 1

For this experiment we employed an established strain (hereinafter referred to as "HRC") originating from a human renal carcinoma.

A growth culture medium comprising Basal Medium Eagle (hereinafter "BME") in which 10 percent new born calf serum was included was first prepared. The growth culture medium was inoculated with HRC cells and the tumour cells were cultivated in a cultivating vessel until a saturation state was reached. The culture medium was then washed once to remove the serum. The cells were then transferred to a culture medium adapted for extraction and including no serum. Glucose was added to samples of the culture medium at a concentration of 1, 2 and 5 grams per litre of the culture medium. Cultivation was carried out at a temperature of 30 to 37°C for a period of one week. For comparative purposes a similar cultivation was carried out in BME in which 10 percent new born calf serum, amino acids and vitamins were included.

Following cultivation, the malignant tumour cells were removed from the medium and the culture medium collected.

We shall now describe the experiments which we have performed to evaluate the inhibiting effect exhibited by the products in accordance with the present invention resulting from the above example.

A BME culture medium in which 10 percent new born calf serum was included was placed in a 15 mm. diameter plastic Petri dish and inoculated with $10^4$ HRC cells. After 24 hours' cultivation in the culture medium, that culture medium was exchanged for an experimental culture medium additionally including the product resulting from the procedure described in Example 1 above.  Replacement with fresh culture medium also including the aforesaid product was carried out every other day.  The extent to which the cells had proliferated was measured on the 6th day.  The control group was evaluated in the same fashion, but with the addition of glucose to its evaluation culture medium at a concentration of 1 gram per litre in order to ensure similar conditions.

The experimental results are set out in Table 1 below.

Table  1

| Experimental Group | | Amount of glucose(g/1) | Number of (x $10^4$ cells/plate) | Survival rate(%) |
|---|---|---|---|---|
| Control | | 1 | 23 | 100 |
| Lot No. 112 | 112-1 | 1 | 11 | 48 |
| | 112-3 | 1 | 12 | 50 |
| | 112-4 | 2 | 4 | 17 |
| | 112-5 | 5 | 2.7 | 12 |

The survival rates given in Table 1 are obtained by regarding the control group as having a survival rate of 100 percent and then representing in terms of percentage the number of cells surviving in the experimental group as a ratio of the number surviving in the control.

It will be seen that the greater the amount of glucose included in the extraction culture medium, the higher the potency of the proliferation inhibitor.  It will be seen that if glucose is added to the extraction culture medium at a concentration of 2 grams per litre or more, the proliferation inhibiting effect is several times greater than in the case of our prior proposal referred to hereinabove, with the consequence that a significant inhibition effect can be obtained in a smaller dose.

Example 2

Glucose was added to BME including 10 percent new born calf serum in a ratio of 5 grams of glucose per litre of BME, and a sub-culture of HRC cells was carried out. The BME was washed once as in the manner described in Example 1 in order to remove the serum and the cells transferred to a culture medium for extraction. After adding glucose to the culture medium, cultivation was performed. The proliferation inhibitor was extracted from the medium in the same manner as described in Example 1.

An evaluation of the proliferation inhibitor produced was carried out in like manner to the evaluation described above for the product of Example 1. The results are set out in Table 2 below.

Table 2

| Experimental Group | | Conc. of glucose (g/l) | Number of cells (x $10^4$ cells/plate) | Survival rate (%) |
|---|---|---|---|---|
| Control | | 1 | 31 | 100 |
| Lot No. 113 | 113-3 | 1 | 10 | 32 |
| | 113-4 | 2 | 5 | 16 |
| | 113-5 | 5 | 4.4 | 14 |

It will again be seen that proliferation inhibitors produced by the process of the present invention have a potency several times greater than proliferation inhibitors produced by our previously proposed process. The higher the concentration of glucose included in the extraction culture medium, the greater is the effect.

CLAIMS

1.    A method for preparing a proliferation inhibitor for malignant tumour cells, characterised in that it comprises the steps of:  cultivating malignant tumour cells; transferring said cells to a culture medium for extraction; adding glucose to said extraction medium; carrying out a cultivation therein; thereafter removing said malignant tumour cells from the culture medium; and collecting said culture medium.

2.    A method according to Claim 1, further characterised in that said malignant tumour cells are derived from an established strain originating from a human renal carcinoma.

3.    A method according to Claims 1 or 2, further characterised in that said culture medium is Basal Medium Eagle.

4.    A method according to any preceding claim, further characterised in that the said step of cultivating malignant tumour cells is performed in a culture medium wherein glucose is included, the cultivated cells being transferred from the said culture medium to the previously mentioned culture medium for extraction.

5.    Whenever produced by a process according to any preceding Claim, a proliferation inhibitor for malignant tumour cells.